# EUROPEAN PATENT SPECIFICATION

(11) **EP 0 794 749 B1**
(45) Date of publication and mention of the grant of the patent: **11.06.2003**
(21) Application number: 95901359.0
(22) Date of filing: 24.11.1994
(51) Int. Cl.: A61F 5/56, A61C 7/08

(54) **A JAW POSITION-REGULATING ORAL DEVICE FOR PREVENTING SNORING**
KIEFERREGULIERUNG ZUR VERMEIDUNG DES SCHNARCHENS
DISPOSITIF DE POSITIONNEMENT DES MACHOIRES POUR L'EVITER LE RONFLEMENT

(30) Priority: 24.11.1993 DK 131593; 07.02.1994 DK 15294; 19.09.1994 DK 107794
(43) Date of publication of application: 17.09.1997
(73) Proprietor: Ingemarsson-Matzen, Natashia, 2500 Valby (DK)
(72) Inventor: INGEMARSSON-MATZEN,Sonnie, DK-2920 Charlottenlund (DK); VOSS, Torsten de, DK-2650 Hvidovre (DK)
(86) International application number: DK9400438
(87) International publication number: WO95014449

(56) References cited:
- EP-A- 0 312 368
- EP-A- 0 337 201
- GB-A- 2 264 868
- US-A- 5 092 346

## Description

### FIELD OF THE INVENTION

The present invention relates to a device adapted to prevent snoring, in particular to prevent or reduce the frequency of snoring and/or Obstructive Sleep Apnea Syndrome (OSAS) during sleep.

### BACKGROUND OF THE INVENTION

OSAS is characterized by respiration arrest caused by occlusion of the pharyngal airways. The severity of OSAS has been described in the medical literature; the syndrome is giving cause to a number of symptoms. Thus, compared to a normal control group without diseases, patients suffering from snoring and/or OSAS appear to have - three times as many cases of coronary heart diseases, - four times as many cerebral illnesses, such as clots, - seven times as many incidents of car accidents and twice as many labour accidents due to day time sleepiness as a result of lack of sleep and/or impaired sleep quality.

Thus, life time expectancy is severely limited for these patients, and their quality of life is compromised.

The fully developed syndrome, OSAS, appears with a frequency of around 1% of the population as a whole; in the age group from 40 years and up, at least 20% are affected by more or less severe manifestations of the snoring syndromes, and of these, up to 80% are males.

Apnea appears when the upper airway passages are being sucked close to the rear part of the throat when the person is trying to breathe during sleep. These are the conditions of apnea which lead to the aggravated symptoms. In this way, the sleep can be interrupted up to as many as 1000 times during the night.

There seems to be evidence that even people who are snoring without any restriction of the breathing are prone to develop cardiovascular and cerebral problems. Furthermore, snoring is regarded as an incipient sign of later manifest sleep apnea conditions.

Apart from the above, the literature describes the following symptoms and diseases:
- General headache
- hypoxic pulmonary vasoconstriction
- cardiomyopathy
- pulmonary hypertonia with cor pulmonale (increased
- pressure in the hear-lung circuits)
- heart arrhythmia
- day time melancholy
- intelligence alterations
- potency disturbances
- in addition to a large number of problems of a more social character, like, e.g., divorce, decreased labour activity, difficulties in keeping conversations in the track due to tiredness, etc.

For the above reasons, it is important to provide devices to eliminate and prevent apnea and the incipient stages thereof.

In the prior art, a number of surgical techniques for removal of the tissue involved in the obstruction have been developed, but all of these techniques seem to incur a certain invalidation of the patient and, at the same time, do not have a fully predictable effect.

Furthermore, a number of medical treatments has been tried out with predominantly deficient or sometimes even damaging effect.

Finally, the scientific literature and the patent literature disclose numerous devices for alarming the snoring patient during sleep; devices for tongue thrust, devices for forward movement of the soft palate; devices for obstructing the oral cavity (delimited by the lips), thereby engaging the sound from the snoring; furthermore, mouth guard-like devices for provocation of either tongue, hyoid bone or jaw position changes, thereby eliminating snoring; - all of these requiring active participation from competent professionals, such as medical doctors, dentists, etc. Among such prior art devices for or attempts to inhibit snoring, the following are of particular interest in the present context:

EP 0 337 201 discloses an orthodontic appliance comprising a first member to engage with the mandibular dentition and a second member to engage with the maxillary dentition. The two members are resiliently hinged together to keep the upper and lower jaw in a normal position.

GB 2 264 868 discloses an anti-snoring device for oral use, comprising members having upper and lower surfaces which engage the user's maxillary and mandibular dental arches respectively. The upper and lower surfaces are spaced so that the mandible is placed in a forwardly offset position relative to its normal position. The spacing also tensions the masticatory muscles to maintain the device in place.

WO 92/11827 discloses an anti-snoring device for oral use consisting of a horseshoe-like upper jaw member for engaging the maxillary dentition, with the downward extending flange intended to extend into the lingual vestibule in order to maintain a forward posture of the lower jaw.

EP O 312 368 discloses an anti-snoring device for oral use which resembles the above-mentioned device, the main difference being the design of the airway passage.

WO 92/05752 discloses an anti-snoring device for oral use consisting of a spatial member congruent with the palate and a lower member adapted to the lingual aspects of the surfaces of the dentition in the lower jaw, hooks being attached to the occlusive plane of the device for fixing the two jaws in a predetermined relation.

WO 90/13276 discloses for correction of tongue thrust and, as a secondary effect, reduction of snoring frequency.

US 5,313,960 discloses an anti-snoring device for oral use consisting of two horseshoe-like individually shaped mouthpiece portions which are connected and fixed in a predetermined position in which the lower jaw protrudes in relation to the upper jaw.

GB 1,569,129 discloses an anti-snoring device for oral use comprising a channel-shaped portion arranged to fit the teeth or gums of the lower jaw and another portion which may be in the form of a flange which is located so as to extend in the front of and in close proximity to the upper teeth or gums when the wearer is awake, engaging the teeth or gums of the other jaw and restricting sagging of the lower jaw and constriction of the air passages in the throat during sleep.

EP O 298 649 discloses an anti-snoring device for oral use consisting of a horseshoe-like member engaging the maxillary dentition and integrated with a mandibular flange positioned between the lower lip and the facial surfaces of the teeth of the lower jaw so as to obstruct the air flow passage through the mouth to the lungs, thereby engaging the snoring.

WO 92/09249 discloses an oral device to reduce or prevent night clenching an grinding of the teeth and snoring, shaped as a shield around the tip of the tongue and placed dorsally to the bimaxillary dentition, with bilateral protrusions on each side of the plica lingualis.

US 4,986,283 and US 5,052,409 disclose devices for reducing tongue thrust and snoring, both apparently being related to WO 90/13276, the common effect being associated with the fact that snoring is impossible during swallowing.

### DISCLOSURE OF THE INVENTION

While the above devices represent attempts to solve the snoring and apnea problems, they are all rather complicated in their design and require the interaction of a professional team in their individual design. Furthermore, they are rather discomfortable for the wearer, and they do not appear convincing with respect to their capability of achieving an effective and long-lasting anti-snoring effect.

Thus, there is a demand for a relatively comfortable device which provides a high degree of inhibitory effect on snoring during even long sleeping periods, such as overnight, without adverse effects on the structures involved, and which at the same time is easy and simple to use and wear for normal non-skilled persons. The present invention provides such a device.

The anti-snore device according to the invention comprises an upper member adapted to engage the maxillary dentition of a human and a lower member adapted to engage the mandibulary dentition of the human, the upper and lower members being resiliently hinged together, wherein the resiliency of the hinging is adapted to allow the physiological movement of the lower jaw in the sagittal plane while retaining a forward position of the lower jaw relative to the upper jaw and thereby keeping the airway passage in the nasopharynx, the oropharynx and the hypopharynx substantially free of occlusion.

The device according to the invention combines two essential functions: the forward positioning of the lower jaw relative to the upper jaw, and the resilient hinging. As will be explained below, the forward positioning of the lower jaw is essential to prevent occlusion of the airway passage in the pharyngeal space during sleep. The resilient hinging makes it possible and realistic to maintain the forward positioning of the lower jaw even during movements in the sagittal plane which unavoidably occur during sleep. This essential combination of features which ensures constant nonconstricted airflow and unrestricted movement in the sagittal plane and thereby ensures a constant efficient function without risk of the device falling out of the mouth of the user and without any substantial discomfort distinguishes the device according to the invention from all of the above-mentioned prior art devices.

It is presently preferred that the force needed to bring the upper and the lower part of the device together when the device is inserted in the mouth is in the range from 0.2 Newton to 20 Newton, preferably from 0.5 Newton to 8 Newton, e.g. from 1 Newton to 4 Newton. The force being exerted at a position of the device corresponding to the position of the foremost incisor or the foremost canine.

The number of teeth that is engaged by the upper and the lower member of the device, respectively, may be different for different embodiments of the present invention. The more teeth that are engaged by the device the deeper into the mouth of the user the device extends and a device extending deep into the mouth can lead to discomfort and even sickness of the user. On the other hand, if only a few teeth are engaged, the device will be unstable in the mouth of the user. The inventors presently prefers that the device extends to the respective centres of the upper first molars when inserted into the mouth of a user.

The device according to the invention may be made of any material, such as metal, alloy, wood, plastics, etc. provided that the device made feels soft and comfortable in the mouth without any constriction or damaging of the tissue, such as gums, tongue, teeth, but at the same time is sufficiently capable of retaining its shape and of exerting a sufficient resiliency towards the muscular tension and forces acted upon the jaws so that it will maintain the lower jaw in the anterior position while allowing normal movements during sleep.

The device according to the invention is preferably made of a resilient non-toxic plastics material, such as a polyvinyl resin, including a vinyl acetate-ethylene copolymer such as poly(ethyl vinyl acetate), or a polyolefin such as polyethylene or polypropylene.

It is particularly preferred that the resilient non-toxic plastics material is a thermoplastic material, such as a cellulose derivative, a vinyl polymer, a polystyrene, a polyamide, an acrylic resin, etc., which can be shaped to adapt to an individual dentition by moderate heating, such as heating to a temperature above normal human body temperature, that is, a temperature of at least 40°C and at the most 80°C, such as at least 50°C and at the most 80°C, e.g. about 70°C. The material presently most preferred by the inventors is ethylene vinyl acetat copolymer.

Preferably, the device according to the present invention may be manufactured by plastics moulding, such as cold moulding, compression moulding, injection moulding, etc. The manufacturing method presently most preferred by the inventors is injection moulding.

The upper and lower members are preferably integrated with each other through resilient hinges made of the same material as the upper and lower members. However, the hinges may be reinforced and their resiliency enhanced by insertion, such as cast in, etc., into-the hinges of a resilient member, such as a resilient plastics member, a metallic resilient member, such as a flat spring, a laminated spring, etc., etc.

A particularly preferred way of shipping the device according to the invention is as a kit comprising the device and a temperature indicator adapted to indicate a temperature change to an elevated temperature at which the material of the device can be shaped. This makes it simple and safe for the end user to mould the device to conform to his or hers specific dentition simply by heating the relevant domain of the device in water the temperature of which is kept in the correct temperature range for the material in question by using the indication of the temperature indicator.

It should be understood that the use of the anti-snore device according to the invention is not limited to prevention or reduction of snoring or OSAS but the device is applicable in any situation where it is desirable to secure free airway passage in human beings, such as during recovery from anaesthesia, during unconsciousness, etc.

In the following, the anti-snoring device aspect of the invention will be explained in further detail with reference to the accompanying drawings is which,
- Figs. 1-5: illustrate the device according to the invention in a relaxed condition (Figs. 1-3) and in an "active" configuration as it will adopt when engaging the upper and lower dentition (Figs. 4 and 5),
- Fig. 6: shows the average shape of the upper (A) and lower (B) jaws, respectively, of an adult human,
- Fig. 7: shows the normal relation between the upper and lower jaws in occlusion,
- Fig. 8: shows the jaw relation when the lower jaw has been positioned downward and forward,
- Fig. 9: shows the anti-snoring device according to the invention in position, causing the lower jaw to adopt a lowered and forward position,
- Fig. 10: shows the relations between the jaws, the tongue and the pharyngeal space of an adult human lying on his back, indicating obstruction in the pharyngeal airways,
- Fig. 11: shows the same human as in Fig. 10 lying on his back, with the device according to the invention in position, indicating freed airway space in the pharyngeal airways,
- Fig. 12: is a diagrammatic representation of the limitations of the movements of the lower jaw in any direction in the sagittal plane, and
- Fig. 13: shows details of the resilient hinges of the device.

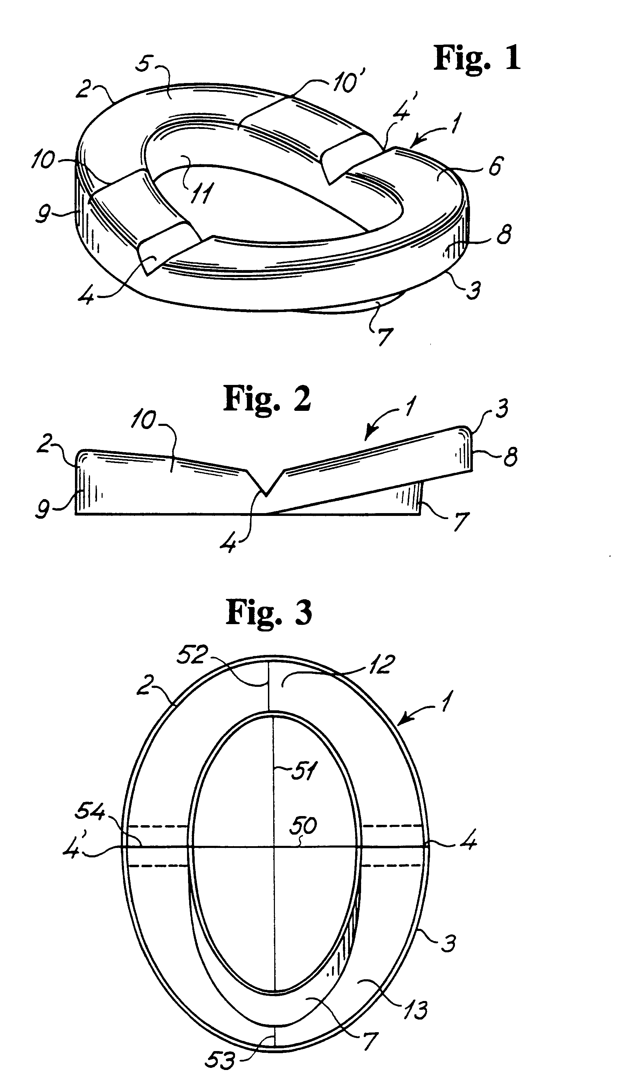

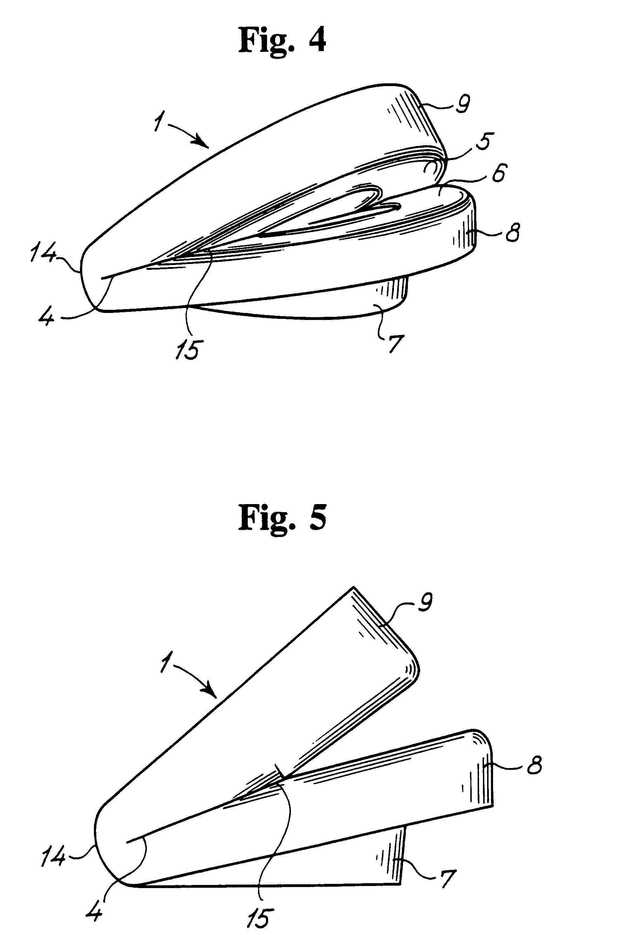

In Figs. 1-5, in which like numerals indicate like parts, the device 1 according to the invention consists of two horseshoe-like members 2 and 3 of a soft, resilient plastics material, preferably a thermoplastic material, such as a ethylene vinyl acetate copolymer hinged together by means of integrated resilient hinges 4 and 4'. Surfaces 5 and 6 represent the lower surface of the upper member and the upper surface of the lower member, respectively. 7 indicates the lingual flange of the lower member adapted to the lingual surfaces of the lower incisors and canines, this flange 7 being the part of the device which actually forces the lower jaw forward. 8 is the facial surface of the lower member, 9 is the facial surface of the upper member, and 11 is the lingual surface of the upper member. The conjoining effect of forces exerted by the facial surface 9 of the upper member and the lingual surface 7 of the lower member keep the lower jaw in a forward position relative to the upper jaw. As the facial surface 8 and the lingual surface 11 do not exert any forces, their dimensions are rather uncritical and some embodiments of the invention may even be provided without these surfaces. 10 and 10' indicate an angulation of the lower surface 5 of the upper member 2, corresponding to the normal curve of the occlusal plane of the upper dentition. Fig. 1 shows the device 1 in perspective. Fig. 2 is a side view of the device 1. Fig. 3 is a bottom view of the device 1, showing a tunnel-shaped space 12 of the upper member 2 adapted to engage the upper dentition and a tunnel-shaped space 13 of the lower member 3 adapted to engage the lower dentition. Figs. 4 and 5 show the device 1 in an activated configuration where the hinges 4 and 4' compressed to form a convexity 14 at the posterior end part of the device and a concavity 15 at the anterior part of the hinging.

Fig. 13 shows details of the resilient hinges 4, 4' of the device 1 where the resilient hinges are made of the same material as the upper and lower members. In this case, the desired resiliency of the hinges is provided by the half-cylinder 40 in cooperation with the notch 41. The numeral 42 marks the diameter of the half-cylinder 40. If this diameter is increased, the force needed to bend the device will increase and, correspondingly, if the diameter is decreased, the resiliency of the device will decrease and eventually the device will not follow the movements of the jaws of a user of the device. Thus, the diameter marked by 42 must be optimized to provide a device with an optimum resiliency, comfortable to wear while still following the jaw movements. The optimum diameter varies for different materials.

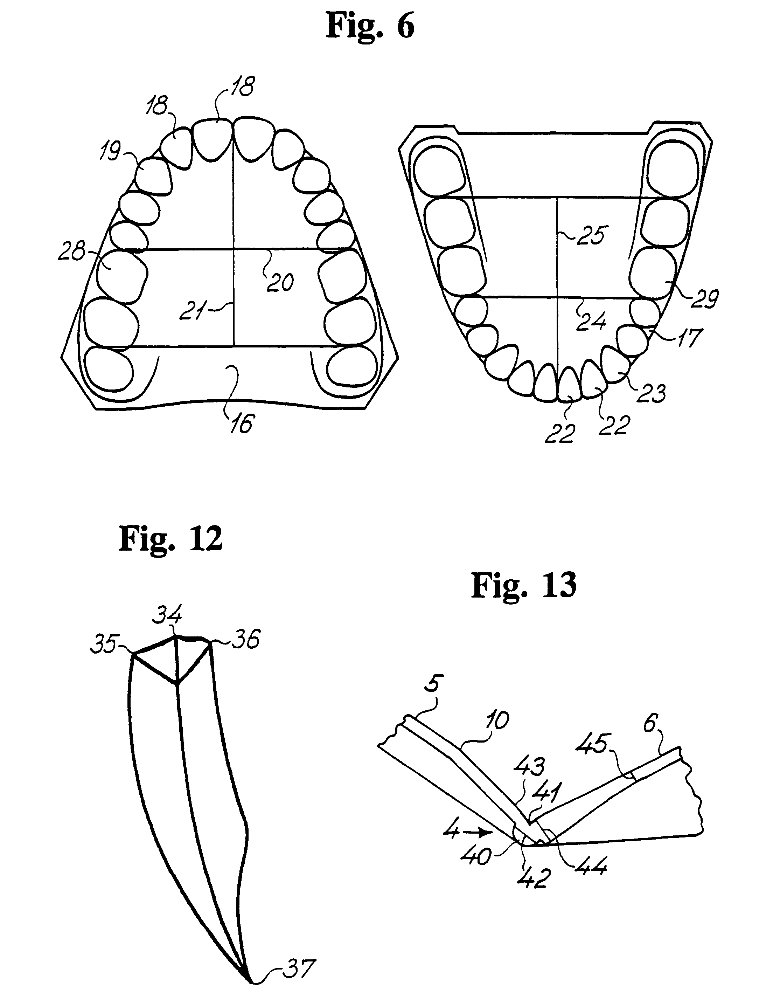

Fig. 6 shows the normal maxilla 16 and the normal mandibula 17 of a human being. In fig. 6 the numeral 18 represents the incisors of the maxilla, 19 represents the canines of the maxilla and numerals 20 and 21 mark the width and the length of the maxilla respectively. The average sizes of the maxilla of a grown up human are 45.5 mm for the width and 48.0 mm for the length. Comparative sizes for the mandibula are 39.6 mm for the width and 44.0 mm for the length of the mandibula where 24 and 25 represent the width and the length respectively, in Fig. 6, and 22 and 23 represent the mandibular incisors and canines respectively. The sizes for both the maxilla and the mandibula are within plus / minus 2.85 mm by one standard deviation. The shape of the jaws is predominantly ovoid for the maxilla, and parabola for the mandibula. Children of different ages will naturally correspond to the same distribution of the teeth as adults, but the size will differ.

Although mouth shapes and sizes vary, a one-size device corresponding to the above-mentioned average sizes of the maxilla and mandibula of a grown up human is provided according to the present invention. A device of such shape conforms with most mouths without individual fitting or with limited individual fitting. However, a range of different sizes of the device may be provided, thereby further minimizing the need for individual fitting. Further, special sizes and shapes of the device may be provided for patients with abnormal dentition, such as mandibula prognatia, etc.

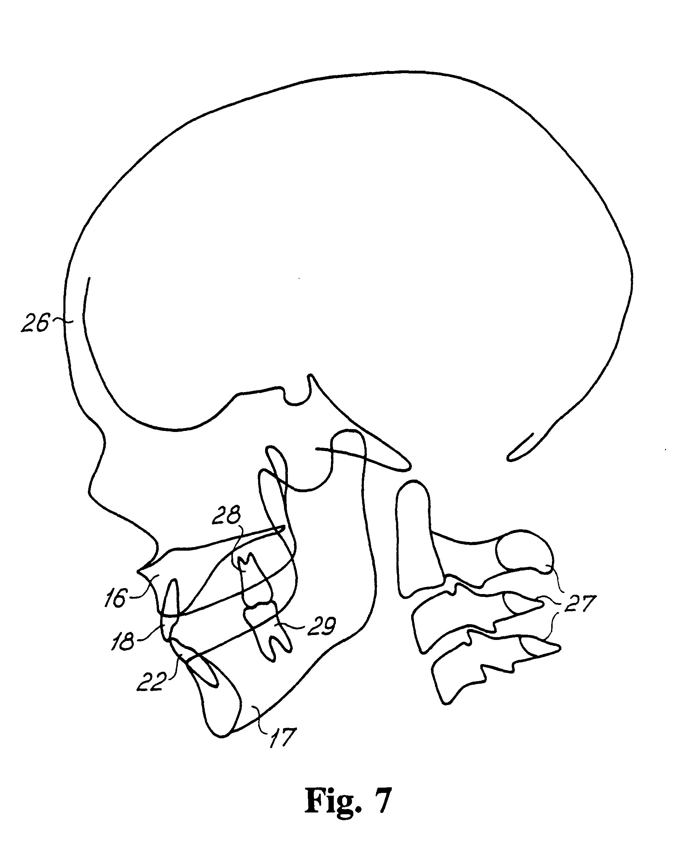

Fig. 7 shows the normal relations between the jaws 16 and 17, the calvaria 26, and the cervical vertebrae 27 of a human. 28 denominates the upper first molar, and 29 denominates the lower first molar.

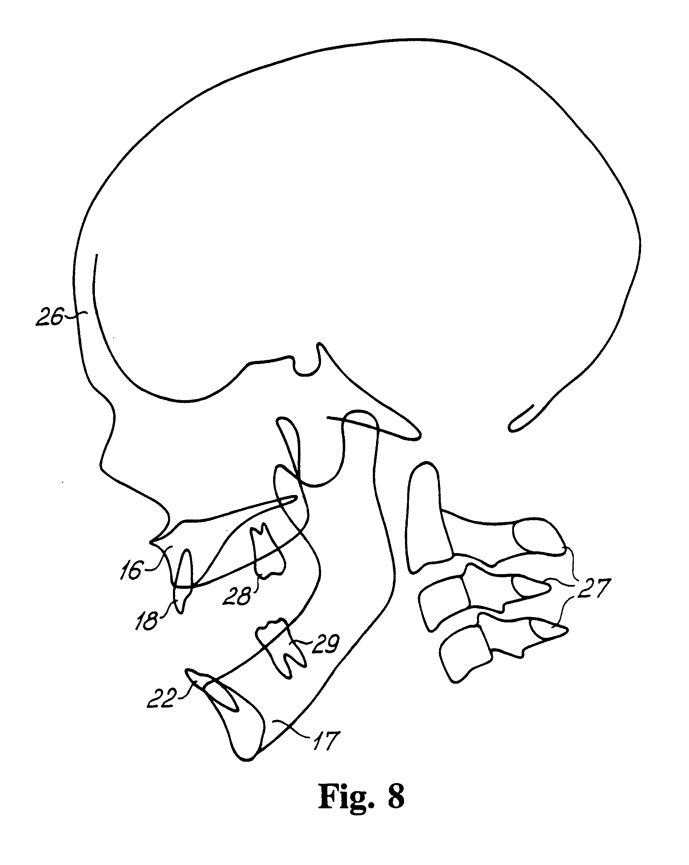

Fig. 8 shows the relations between the jaws 16 and 17, the calvaria 26, and the cervical vertebrae 27 of a human when the device according to the invention is being used, thereby displacing the lower jaw in a protruded and caudally placed position.

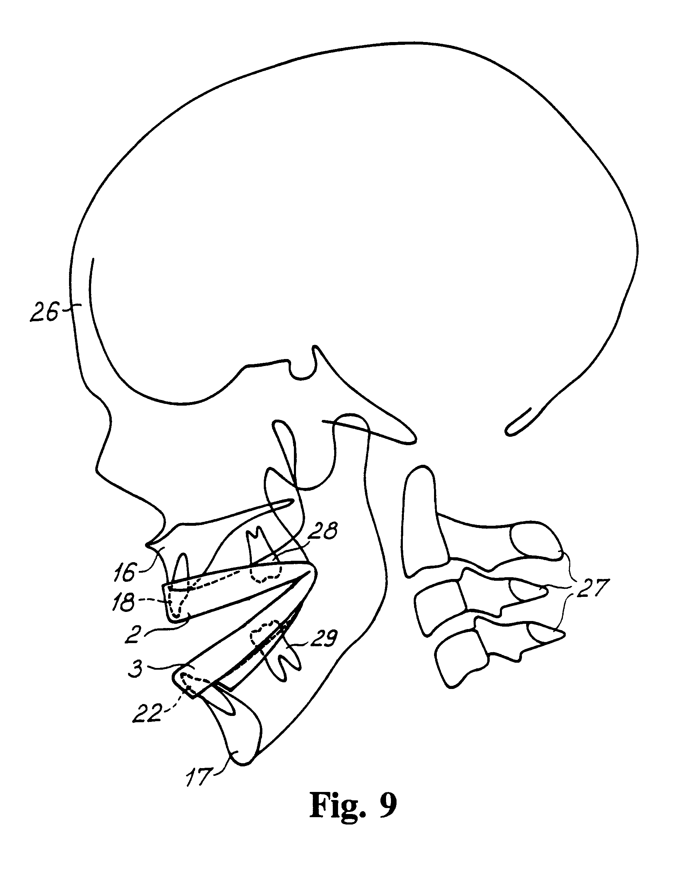

Fig. 9 shows the invention, with the upper member 2 and the lower member 3 hinged together, positioned between the jaws of a human, thereby showing the protruded and caudally placed position of the lower jaw.

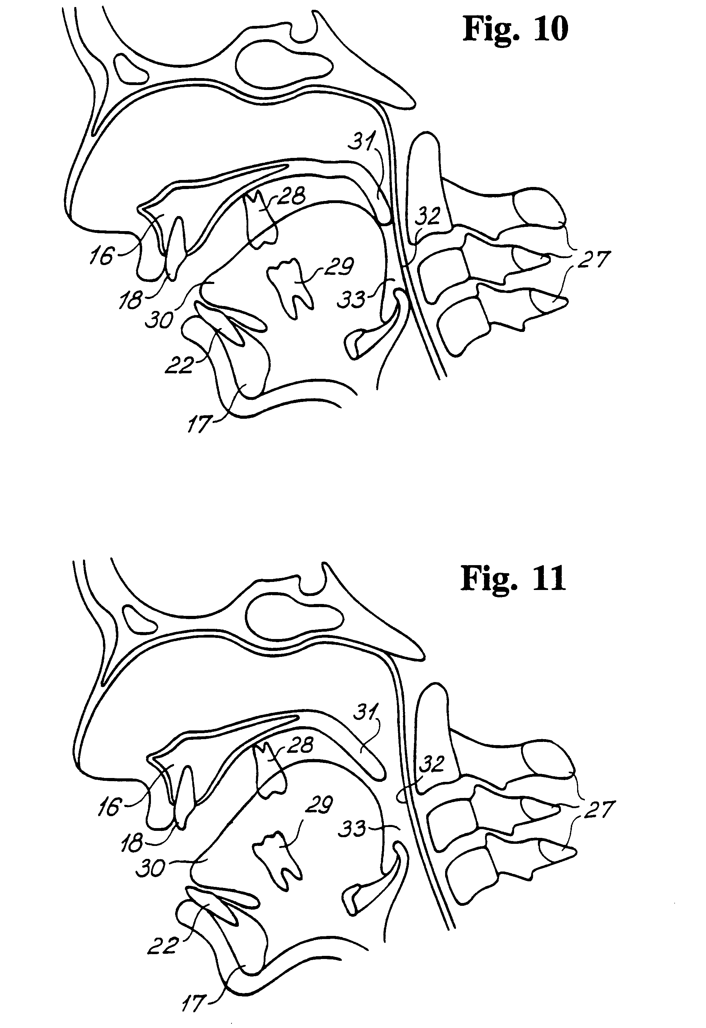

Fig. 10 shows the normal relations between the oro-pharyngeal constituents of a human in relaxed position during sleep in a lying position; 30 denominates the tongue, 31 the uvula, 32 the dorsal pharyngeal wall, and 33 the pharyngeal airway space.

Fig. 11 shows the relations between the oro-pharyngeal constituents of a human when he is using the device according to the invention.

Fig. 12 shows the outer contours for the movement of the mandible of a normal human in the sagittal plane; 34 is the intercupidal position (IP) in which the dentition of the mandible makes the maximal interference with the dentition of the maxilla; 35 is the protruded contact position (PCP) in which the mandible has made the maximal protruded movement from the IP position, still keeping some contact with the dentition of the maxilla; 36 is the retracted contact position (RCP) in which the mandible have made the maximal retraction from the IP position, still keeping some contact with the dentition of the maxilla, and 37 is the maximal opening point (MOP) in which the mandible has made the maximal opening movement from the IP position, all of which only being restricted by the muscles, the teeth, the ligaments and the discus involved in the temporomandibular joint system.

As will be understood the devices of the prior art all seem to show deficiencies with respect to keeping the mandible in a forward position relative to the maxilla even during movements of the mandible like yawning during sleep, thereby eliminating the effect otherwise obtained from the devices known to day. For instance, the device mentioned in WO 92/11827 would seem to be unable to function because the biomechanical feedback mechanism of the dentoalveolartemporomandibular proprioceptive nerve threads receptors will direct the mandible to fall down when touching the pivot-like part of the lower portion of the device in question.

During sleep, most people are lying on their back. Figure 10 illustrates what happens when a human is lying on the back during sleep: the mandible is falling back in the posterior part of the pharynx, thereby occluding the free airway space needed to provide the lungs with sufficient amount of oxygenated air. This is illustrated in Fig. 12 where the movement of the lower jaw is characterized by the space described by the movement of the most cranial part of the central incisor of the mandible (incisivus inferior) in the sagittal plane in any outer limiting position only restricted by anatomic structures of the temporomandibular joint. The movement of the lower jaw are following the outer contours of Fig.12 when the mandible is moving along its most extreme borders going backwards, the mandible reaches the retracted contact position (RCP), still keeping contact with the maxillary dentition, from where it runs along the posterior line between RCP and MOP (the maximum opening point). Leaving the MOP anteriorly, the incisivus inferius runs along the line between MOP and PCP (the protruded contact position) again achieving contact with the dentition of the maxilla in the most forward placed position of the mandible. The present invention aims at keeping the mandible (described by the movements of the incisivus inferius in the sagittal plane) in the space outlined by PCP-IP-MOP as illustrated in Fig. 11, thus keeping the airway in the pharynx free of any occlusion, thereby eliminating snoring and the resultant sleep apnea syndrome.

Furthermore, it is an advantageous feature of the device according to the present invention that the end user does not need assistance from competent professionals, such as medical doctors, dentists, etc. to be able to adapt the device to his or hers specific dentition and to use the device. Thus, the patient saves time and money.

### EXAMPLE

A one-size device has been manufactured from ethylene vinyl acetate copolymer by injection moulding with the approximate dimensions disclosed below.

The material thickness differs for different regions of the device but the average thickness is app. 2 mm. The length marked by numeral 50 is app. 56 mm and the width marked by numeral 51 is app. 30 mm. The width marked by numeral 52 is app. 7 mm, the width marked by numeral 53 is app. 4 mm, and the width marked by numeral 54 is app. 12 mm. The angle at the position marked by numeral 10 is app. 10° corresponding to the curve of the occlusal plane (Spee's curve), and the angle between the facial surface 8 and the surface 6 is app. 85°, and the angle between the lingual flange 7 and the surface 6 is app. 85°. The angle between the facial surface 9 and the surface 5 is app. 85°, and the angle between the lingual surface 11 and the surface 5 is app. 85°. The heights of the flanges 7, 8, 9, 11 at the anterior part of the device when inserted in a mouth are app. 10 mm, 7 mm, 10 mm, and 7 mm, respectively, measured from the lower surface of the lower member and the upper surface of the upper member, respectively. The diameter marked by numeral 42 is app. 4 mm.

The vertex angle of the notch 41 is app. 90° when no forces are exerted on the device. The angle at the position marked by numeral 43 is app. 7° and the distance from the position marked by numeral 43 to the vertex of the notch is app. 3 mm. The distance between the positions marked by numerals 10 and 43 is app. 19 mm. The thickness of the material is app. 4 mm at the position marked by numeral 44 decreasing gradually to app. 2 mm at the position marked by numeral 45. The distance between the positions marked by numerals 44 and 45 is app. 20 mm.

This device has been tested on 25 patients with snoring problems. During this study, the width of the airway passage (figs. 10 and 11) of each patient was measured with the patient lying on his or hers back with and without the device inserted in the mouth. X-ray cephalograms of the airway passage was used for the measurements. The width of the airway passage was measured at seven different points in the passage and added to a sum. On average this sum was approximately 125 mm with no device inserted in the mouth and approximately 140 mm with the device inserted in the mouth. Thus, a significant increase in the width of the passage was obtained with the device inserted.

Further, in a questionnaire, 19 of 20 patients reported a significant improvement in sleep quality when they used the device.

## Claims

1. An anti-snore device (1) comprising an upper member (2) adapted to engage the maxillary dentition (18, 19, 28) of a human and a lower member (3) adapted to engage the mandibulary dentition (22, 23, 29) of the human, **characterized in that** the upper and lower members (2, 3) are resiliently hinged together, whereby the resiliency of the hinging is adapted to allow the physiological movement of the lower jaw (17) in the sagittal plane while retaining a forward position of the lower jaw (17) relative to the upper jaw (16) and thereby keeping the airway passage in the nasopharynx, the oropharynx and the hypopharynx substantially free of occlusion.

2. A device (1) according to claim 1, which is adapted to keep the lower jaw (17) in the positions corresponding to the area between the intercuspidal position (34), the protruded contact position (35), and the maximum opening point of the jaw (37).

3. A device (1) according to claim 2, which is adapted to keep the lower jaw (17) substantially in the positions corresponding to the anterior border of the physiological space of movement of the lower jaw (17) as limited by the anatomical structures in the temporo mandibular joints.

4. A device (1) according to claim 3, wherein the forward displacement of the lower jaw (17) about 22 mm from the intercuspidal position along the IP-MOP curve is in the range between about 5 and about 11 mm.

5. A device (1) according to any of the preceding claims, wherein the upper member (2) is a substantially horseshoe-formed member having an anterior wall adapted to be in contact with the facial surfaces of the incisors and/or canines of the upper jaw (16) and the lower member (3) is a substantially horseshoe-formed member having a posterior wall adapted to be in contact with the lingual surface of the incisors and/or canines of the lower jaw (17).

6. A device (1) according to claim 5, wherein the anterior wall of the upper member (2) is ellipsoid substantially in accordance with the normal human dentition, and the posterior wall of the lower member (3) is shaped as a parabola substantially in accordance with the normal human dentition.

7. A device (1) according to claim 5 or 6, wherein the upper and lower members (3) have bases substantially conforming with the occlusal plane of a human.

8. A device (1) according to claim 7, wherein the upper member (2) has a posterior wall positioned palatinally to the incisors and/or canines of the upper jaw (16), and the lower member (3) has an anterior wall positioned facially to the incisors and/or canines of the lower jaw (17).

9. A device (1) according to any of the preceding claims, which is made of a resilient non-toxic plastics material, such as a polyvinyl resin, including a vinyl acetate-ethylene copolymer such as poly(ethyl vinyl acetate), or a polyolefin such as polyethylene or polypropylene.

10. A device (1) according to claim 9, wherein the resilient non-toxic plastics material is a thermoplastic material which can be shaped to adapt to an individual dentition by moderate heating.

11. A device (1) according to claim 10, wherein the temperature at which the material can be shaped is at least 40°C and at the most 80°C, such as at least 50°C and at the most 80°C, e.g. about 70°C.

12. A device (1) according to claim 10 or 11, wherein the upper and lower members (2, 3) are integrated with each other through resilient hinges made of the same material as the upper and lower members (2, 3).

13. A device (1) according to claim 10 or 11, wherein the upper and lower members (2, 3) are integrated with each other through resilient hinges that are reinforced and their resiliency enhanced by insertion of a resilient member.

14. A device (1) according to claim 12 or 13, wherein the hinges are constituted by domains which are convex in the posterior corners of the device (1) and concave in the anterior angle of the device (1) when positioned in the mouth.

15. A kit comprising a device (1) according to any of claims 10-14 together with a temperature indicator adapted to indicate a temperature change to an elevated temperature at which the material of the device (1) can be shaped.

## Patentansprüche

1. Anti-Schnarch-Vorrichtung (1), bestehend aus einem Oberteil (2), das auf die maxilläre Zahnreihe (18, 19, 28) eines Menschen paßt, und einem Unterteil (3), das auf die mandibuläre Zahnreihe (22, 23, 29) des Menschen paßt. Ober- und Unterteil (2, 3) sind durch ein elastisches Scharnier miteinander verbunden; die Elastizität des Scharniers ist **dadurch gekennzeichnet, daß** es den physiologischen Bewegungen des Unterkiefers (17) auf der Sagittalebene unter Beibehaltung der Vorverlagerung des Unterkiefers (17) in Relation zum Oberkiefer (16) folgt und dadurch die Atemwege im Nasopharynx, Oropharynx und Hypopharynx weitgehend von Obstruktionen freihält.

2. Vorrichtung (1) nach Anspruch 1, **dadurch gekennzeichnet, daß** der Unterkiefer (17) in Positionen gehalten wird, die dem Bereich zwischen der Interkuspidalposition (34), der vorgelagerten Kontaktposition (35) und dem maximalen Öffnungspunkt des Kiefers (37) entsprechen.

3. Vorrichtung (1) nach Anspruch 2, **dadurch gekennzeichnet, daß** der Unterkiefer (17) im wesentlichen in Positionen gehalten wird, die der vorderen Grenze des physiologischen Bewegungsradius' des Unterkiefers (17) entsprechen, der durch die anatomischen Strukturen in den Unterkiefergelenken begrenzt wird.

4. Vorrichtung (1) nach Anspruch 3, **dadurch gekennzeichnet, daß** die Vorverlagerung des Unterkiefers (17) etwa 22 mm von der Interkuspidalposition entlang der IP-MOP-Kurve im Bereich zwischen 5 mm und etwa 11 mm liegt.

5. Vorrichtung (1) nach einem der vorangehenden Ansprüche, **dadurch gekennzeichnet, daß** das Oberteil (2) ein weitgehend hufeisenförmiges Teil mit einer anterioren Wand ist, die auf der Fazialoberfläche der Schneidezähne und/oder der Eckzähne des Oberkiefers (16) aufliegt, und das Unterteil (3) ein weitgehend hufeisenförmiges Teil mit einer posterioren Wand ist, die auf der Lingualoberfläche der Schneidezähne und/oder der Eckzähne des Unterkiefers (17) aufliegt.

6. Vorrichtung (1) nach Anspruch 5, **dadurch gekennzeichnet, daß** die anteriore Wand des Oberteils (2) in weitgehender Übereinstimmung mit dem normalen menschlichen Gebiß ellipsenförmig ist, und die posteriore Wand des Unterteils (3) in weitgehender Übereinstimmung mit dem normalen menschlichen Gebiß parabolförmig ist.

7. Vorrichtung (1) nach Anspruch 5 oder 6, **dadurch gekennzeichnet, daß** die Grundplatten des Ober- und Unterteils (3) weitgehend der Okklusionsebene eines Menschen entsprechen.

8. Vorrichtung (1) nach Anspruch 7, **dadurch gekennzeichnet, daß** das Oberteil (2) mit einer posterioren Wand ausgestattet ist, die palatal zu den Schneide- und/oder den Eckzähnen des Oberkiefers (16) positioniert ist, und das Unterteil (3) mit einer anterioren Wand ausgestattet ist, die fazial zu den Schneide- und/oder den Eckzähnen des Unterkiefers (17) positioniert ist.

9. Vorrichtung (1) nach einem der vorangehenden Ansprüche, **dadurch gekennzeichnet, daß** sie aus elastischem, ungiftigem Kunststoffmaterial wie Polyvinylharz, einschließlich einem Vinylacetatethylen-Copolymer wie Poly(ethylvinylacetat) oder einem Polyolefin wie Polyethylen oder Polypropylen, besteht.

10. Vorrichtung (1) nach Anspruch 9, **dadurch gekennzeichnet, daß** das ungiftige Kunststoffmaterial ein thermoplastisches Material ist, das durch moderate Erwärmung so geformt werden kann, daß es sich einem individuellen Gebiß anpaßt.

11. Vorrichtung (1) nach Anspruch 10, **dadurch gekennzeichnet, daß** die Temperatur, bei der das Material geformt werden kann, mindestens 40 °C und höchstens 80 °C beträgt, so daß mindestens 50 °C und höchstens 80 °C, also beispielsweise etwa 70 °C, notwendig sind.

12. Vorrichtung (1) nach Anspruch 10 oder 11, **dadurch gekennzeichnet, daß** das Oberund das Unterteil (2, 3) durch elastische Scharniere miteinander verbunden sind, die aus dem gleichen Material angefertigt sind, wie das Ober- und Unterteil (2, 3).

13. Vorrichtung (1) nach Anspruch 10 oder 11, **dadurch gekennzeichnet, daß** das Oberund das Unterteil (2, 3) durch elastische Scharniere miteinander verbunden sind, die verstärkt wurden und deren Elastizität durch die Integration eines elastischen Teils vergrößert wurde.

14. Vorrichtung (1) nach Anspruch 12 oder 13, **dadurch gekennzeichnet, daß** die Scharniere aus Teilen bestehen, die, wenn im Mund plaziert, an den hinteren Enden der Vorrichtung (1) konvex und am vorderen Winkel der Vorrichtung (1) konkav geformt sind.

15. Ein Set, das aus einer Vorrichtung (1) nach einem der vorangehenden Ansprüche 10 bis 14 und einer Temperaturanzeige besteht, die die Temperaturveränderung auf eine erhöhte Temperatur anzeigt, bei der das Material der Vorrichtung (1) geformt werden kann.

## Revendications

1. Un dispositif anti-ronflement (1), comprenant une partie supérieure (2) adaptée à engager la dentition maxillaire (18, 19, 28) d'un humain, et une partie inférieure adaptée à engager la dentition mandibulaire (22, 23, 29) de l'humain, les deux parties (2, 3) étant jointes par une charnière résiliente permettant le mouvement psysiologique de la mâchoire inférieure (17) au plan sagittal tout en maintenant une position avancée (17) par rapport à la mâchoire supérieure et ainsi empêcher l'occlusion des nasopharynx, oropharynx et hypopharynx de la voie respiratoire.

2. Un dispositif (1), conforme à la revendication 1, adapté à tenir la mâchoire inférieure (17) dans les positions correspondantes à la zone entre la position intercuspidale (34), la position de contact avancée (35) et la position d'ouverture maximale de la mâchoire (37).

3. Un dispositif (1), conforme à la revendication 2, adapté à tenir solidement la mâchoire inférieure (17) dans les positions correspondantes à la bordure antérieure de l'espace psysiologique de mouvement de la mâchoire inférieure (17) comme limité par les structures anatomiques des joints temporo mandibulaires.

4. Un dispositif (1), conforme à la revendication 3, dont le déplacement avancé de la mâchoire inférieure (17) environ 22 mm de la position intercuspidale au long de la courbe IP-MOP se trouve dans l'écart entre approximativement 5 et 11 mm.

5. Un dispositif (1), conforme aux les revendications précédents, dont la partie supérieure (2), formée comme un fer à cheval et ayant un rebord antérieur, est adapté à être en contact avec les surfaces faciales des incisives et/ou des canines de la mâchoire supérieure (16), et dont la partie inférieure (3), également formée comme un fer à cheval et ayant un rebord posterieur adapté à être en contact avec la surface linguale des incisives et/ou des canines de la mâchoire inférieure (17).

6. Un dispositif (1), conforme à la revendication 5, dont le rebord antérieur de la partie supérieure (2) est ellipsoïde en conformité de la dentition normale des humains, et le rebord postérieur de la partie inférieure (3) de forme paraboloïde en conformité de la dentition normale des humains.

7. Un dispositif (1), conforme à les revendications 5 ou 6, dont la partie inférieure et la partie supérieure (3) ont des bases conformes au plan occlusif de l'humain.

8. Un dispositif (1), conforme à la revendication 7, dont la partie supérieure (2) a un rebord postérieur placé de manière palatale par rapport aux incisives et/ou canines de la mâchoire supérieure (16) et dont la partie inférieure (3) a un rebord antérieur placé face aux incisives et/ou canines de la mâchoire inférieure (17).

9. Un dispositif (1), conforme aux les revendications précédents, fait de matériaux résilients plastiques non-toxiques c.-à-d. résine vinylique, acetate-ethylene copolymère vinylique comme le poly(ehtyl vinyl acétate), ou une polyoléfine comme le polyéthylène ou le polypropylène.

10. Un dispositif (1), conforme à la revendication 9, dont les matériaux résilients plastique non-toxiques sont thermoplastiques ce qui permet de l'adapter aux dentitions individuelles par réchauffement modéré.

11. Un dispositif (1), conforme à la revendication 10, dont la température nécessaire pour changer l'aspect du dispositif est supérieure à 40°C et inférieure à 80°C, comme par exemple 50°C au minimum et 80°C au maximum, ou bien environ 70°C.

12. Un dispositif (1), conforme à les revendications 10 ou 11, dont la partie inférieure et la partie supérieure (2, 3) sont intégrées l'une dans l'autre par des charnières résilientes faites des mêmes matériaux que les parties inférieure et supérieure (2, 3).

13. Un dispositif (1), conforme à les revendications 10 ou 11, dont la partie inférieure et la partie supérieure (2, 3) sont intégrées l'une dans l'autre par des charnières résilientes renforcées et dont la résilience a été renforcée par l'installation d'une unité résiliente.

14. Un dispositif (1), conforme à les revendications 12 ou 13, dont les charnières sont constituées de domaines qui sont convexes dans les encoignures postérieures du dispositif (1), et concaves dans l'angle antérieure du dispositif (1) lorsque celui-ci est placé dans la bouche.

15. Une ensemble comprenant un dispositif (1) conforme aux revendications 10-14, accompagné d'un thermomètre adapté à indiquer un changement de température à un niveau suffisamment élévé pour modifier les matériaux du dispositif (1).
